# EUROPÄISCHE PATENTANMELDUNG

(11) **EP 3 508 470 A1**
(43) Veröffentlichungstag der Anmeldung: **10.07.2019**
(21) Anmeldenummer: 18150230.3
(22) Anmeldetag: 03.01.2018
(51) Int. Cl.: C07C 2/84, C07C 5/48, C07C 9/06, C07C 11/04, C10G 9/36

(54) **VERFAHREN UND ANLAGE ZUR GEWINNUNG EINES ODER MEHRERER OLEFINE**

(71) Anmelder: Linde Aktiengesellschaft, 80331 München (DE)
(72) Erfinder: Winkler, Florian, 81241 München (DE); Walter, Dr. Stephanie, 82418 Seehausen (DE); Nold, Dr. Michael, 82515 Wolfratshausen (DE); Pöschl, Matthias, 85635 Höhenkirchen-Siegertsbrunn (DE)
(74) Vertreter: m patent group

(57) **Zusammenfassung**

Die Erfindung betrifft ein Verfahren zur Gewinnung eines oder mehrerer Olefine, bei dem ein Methan und Sauerstoff enthaltendes Einsatzgemisch (1) einer oder mehreren katalytischen Zonen (11) eines oder mehrerer Reaktoren (10) zugeführt werden, wobei die eine oder die mehreren katalytischen Zonen (11) einen Katalysator zur oxidativen Kopplung von Methan aufweisen, wobei der einen oder den mehreren katalytischen Zonen (11) ein Gasgemisch entnommen wird und zumindest teilweise in eine oder mehrere nichtkatalytische Zonen (12) des einen oder der mehreren Reaktoren (10) überführt wird, wobei in die eine oder die mehrere nichtkatalytischen Zonen (12) ein oder mehrere Paraffine eingespeist werden, und wobei das eine oder die mehreren in die eine oder in die mehreren nichtkatalytischen Zonen (12) eingespeisten Paraffine in der einen oder in den mehreren nichtkatalytischen Zonen (12) teilweise durch eine Dampfspaltreaktion zu einem oder mehreren Olefinen umgesetzt werden. Es ist vorgesehen, dass das eine oder die mehreren Paraffine an einer oder an mehreren von mehreren unterschiedlichen Einspeisepositionen (13a, 13b, 13c), die während der Durchführung des Verfahrens in Abhängigkeit von einer vorgegebenen Konversion des einen oder der mehreren Paraffine ausgewählt wird oder werden, in die eine oder die mehreren nichtkatalytischen Zonen (12) eingespeist werden. Eine entsprechende Anlage (100) ist ebenfalls Gegenstand der Erfindung.

## Beschreibung

Die Erfindung betrifft ein Verfahren zur Gewinnung eines oder mehrerer Olefine und eine entsprechende Anlage gemäß den Oberbegriffen der jeweiligen unabhängigen Patentansprüche.

### Stand der Technik

Die oxidative Kopplung von Methan ist in der Literatur beschrieben, beispielsweise bei J.D. Idol et al., "Natural Gas", in: J.A. Kent (Hrsg.), "Handbook of Industrial Chemistry and Biotechnology", Band 2, 12. Auflage, Springer, New York 2012.

Die oxidative Kopplung von Methan umfasst nach derzeitigem Kenntnisstand eine katalysierte Gasphasenreaktion von Methan mit Sauerstoff, bei der von zwei Methanmolekülen jeweils ein Wasserstoffatom abgespalten wird. Die dabei entstehenden Methylradikale reagieren zunächst zu einem Ethanmolekül. Bei der Reaktion wird ferner ein Wassermolekül gebildet. Bei geeigneten Verhältnissen von Methan zu Sauerstoff, geeigneten Reaktionstemperaturen und der Wahl geeigneter Katalysebedingungen erfolgt anschließend eine Oxydehydrierung des Ethans zu Ethylen, einer Zielverbindung bei der oxidativen Kopplung von Methan. Hierbei wird ein weiteres Wassermolekül gebildet. Der eingesetzte Sauerstoff wird bei den genannten Reaktionen typischerweise vollständig umgesetzt.

Die Reaktionsbedingungen bei der oxidativen Kopplung von Methan umfassen klassischerweise eine Temperatur von 500 bis 900 °C, einen Druck von 5 bis 10 bar und hohe Raumgeschwindigkeiten. Jüngere Entwicklungen gehen insbesondere auch in Richtung der Verwendung tieferer Temperaturen. Die Reaktion kann homogen- und heterogenkatalytisch im Festbett oder in der Wirbelschicht erfolgen. Bei der oxidativen Kopplung von Methan können auch höhere Kohlenwasserstoffe mit bis zu sechs oder acht Kohlenstoffatomen gebildet werden, der Schwerpunkt liegt jedoch auf Ethan bzw. Ethylen und ggf. noch Propan bzw. Propylen.

Insbesondere aufgrund der hohen Bindungsenergie zwischen Kohlenstoff und Wasserstoff im Methanmolekül sind die Ausbeuten bei der oxidativen Kopplung von Methan vergleichsweise gering. Typischerweise werden nicht mehr als 10 bis 15% des eingesetzten Methans umgesetzt. Außerdem begünstigen die vergleichsweise harschen Reaktionsbedingungen und Temperaturen, die zur Spaltung dieser Bindungen erforderlich sind, auch die weitere Oxidation der Methylradikale und anderer Intermediate zu Kohlenmonoxid und Kohlendioxid.

Wenngleich den geringen Ausbeuten und der Bildung von Kohlenmonoxid und Kohlendioxid teilweise durch die Wahl optimierter Katalysatoren und angepasster Reaktionsbedingungen entgegengewirkt werden kann, enthält ein bei der oxidativen Kopplung von Methan gebildetes Gasgemisch neben den Zielverbindungen wie Ethylen und ggf. Propylen überwiegend nicht umgesetztes Methan sowie Kohlendioxid, Kohlenmonoxid und Wasser. Durch ggf. erfolgende nichtkatalytische Spaltreaktionen können auch beträchtliche Mengen an Wasserstoff enthalten sein. Ein derartiges Gasgemisch wird im hier verwendeten Sprachgebrauch auch als "Produktgemisch" der oxidativen Kopplung von Methan bezeichnet, obwohl es überwiegend nicht die gewünschten Produkte, sondern auch das nicht umgesetzte Edukt Methan und die soeben erläuterten Nebenprodukte enthält.

Bei der oxidativen Kopplung von Methan können Reaktoren eingesetzt werden, in denen einer katalytischen Zone eine nichtkatalytische Zone nachgeschaltet ist. Das aus der katalytischen Zone ausströmende Gasgemisch wird in die nichtkatalytische Zone überführt, wo es zunächst noch auf den vergleichsweise hohen Temperaturen vorliegt, die in der katalytischen Zone eingesetzt werden. Insbesondere durch die Anwesenheit des bei der oxidativen Kopplung von Methan gebildeten Wassers ähneln die Reaktionsbedingungen hier jenen herkömmlicher Dampfspaltverfahren (engl. Steam Cracking). Daher können hier Ethan und höhere Paraffine zu Olefinen umgesetzt werden. In die nichtkatalytische Zone können auch weitere Paraffine eingespeist werden, so dass die Restwärme der oxidativen Kopplung von Methan in besonders vorteilhafter Weise ausgenutzt werden kann. Ein derartiges gezieltes Dampfspalten in einer der katalytischen Zone nachgeschalteten nichtkatalytischen Zone wird auch als "Post Bed Cracking" bezeichnet. Nachfolgend wird hierfür auch der Begriff "postkatalytisches Dampfspalten" verwendet.

Bezüglich den beim Dampfspalten herrschenden Reaktionsbedingungen sei ebenfalls auf Fachliteratur wie den Artikel "Ethylene" in Ullmann's Encyclopedia of Industrial Chemistry, Onlineausgabe, 15. April 2007, DOI 10.1002/14356007.a10_045.pub2, verwiesen. Das Dampfspalten wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Die vorliegende Erfindung stellt sich die Aufgabe, Verfahren und Anlagen, in denen eine oxidative Kopplung von Methan und ein postkatalytisches Dampfspalten eingesetzt wird, zu verbessern.

### Offenbarung der Erfindung

Vor diesem Hintergrund schlägt die vorliegende Erfindung ein Verfahren zur Gewinnung eines oder mehrerer Olefine und eine entsprechende Anlage gemäß den Oberbegriffen der jeweiligen unabhängigen Patentansprüche vor. Ausgestaltungen sind jeweils Gegenstand der abhängigen Patentansprüche sowie der nachfolgenden Beschreibung.

Vor der Erläuterung der Merkmale und Vorteile der vorliegenden Erfindung werden noch einige Grundlagen und die verwendeten Begriffe erläutert.

Flüssige und gasförmige Gemische können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 1 0%, 5%, 1%, 0,1% oder 0,01 % auf molarer, Gewichts- oder Volumenbasis stehen kann. Der Begriff "überwiegend" kann der Definition von "reich" entsprechen.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, deren Maximal- und Minimalwerte sich um beispielsweise nicht mehr als 1%, 5%, 10%, 20% oder sogar 50% unterscheiden.

Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich jeweils um Absolutdrücke.

### Vorteile der Erfindung

Wie bereits erwähnt, können bei der oxidativen Kopplung von Methan Reaktoren eingesetzt werden, in denen einer katalytischen Zone eine nichtkatalytische Zone nachgeschaltet ist. Die vorliegende Erfindung ist dabei explizit nicht auf die Verwendung mit nur einem Reaktor beschränkt. Beispielsweise kann auch ein Gas oder Gasgemisch in mehreren katalytischen Zonen eines oder mehrerer Reaktoren gebildet und in eine oder mehrere nichtkatalytische Zonen eines oder mehrerer (derselben oder optional weiterer) Reaktoren überführt werden. Beliebige Anordnungen sind möglich. Jedoch wird hierbei dem aus dem einen oder den mehreren katalytischen Zonen in die eine oder in die mehreren nichtkatalytischen Zonen strömenden Gasgemisch keine zusätzliche Wärme zugeführt.

Das aus der einen oder den mehreren katalytischen Zonen ausströmende Gasgemisch wird in die eine oder die mehreren nichtkatalytischen Zonen überführt, wo es, wie erwähnt, aufgrund der hier vorliegenden Reaktionsbedingungen zu Reaktionen kommen kann, die jenen herkömmlicher Dampfspaltverfahren ähneln oder gleichen. Die Reaktionen in der einen oder in den mehreren katalytischen Zonen liefern dabei im Idealfall einen konstanten Einsatzstrom, der zumindest teilweise der einen oder den mehreren nichtkatalytischen Zonen zugeführt werden kann. In die eine oder in die mehreren nichtkatalytischen Zonen können ferner zusätzlich extern bereitgestellte oder rückgeführte Paraffine wie insbesondere Ethan eingebracht werden, um die Ausbeute des Gesamtverfahrens an Olefinen wie Ethylen steigern und die latente Wärme aus der katalytischen Reaktion, d.h. der oxidativen Kopplung, von typischerweise 850 °C bis zu 920 °C nutzen zu können.

Da die für die nichtkatalytischen Reaktionen genutzten Reaktoren bzw. die nichtkatalytische Zone oder Zonen eines oder mehrerer Reaktoren, die für das postkatalytische Dampfspalten eingesetzt werden, typischerweise Rohre mit fester Geometrie darstellen, und da die Menge des aus der einen oder den mehreren katalytischen Zonen in die eine oder die mehreren nichtkatalytischen Zonen abströmenden Gasgemischs, das die Wärme zum Dampfspalten bereitstellt, in der Regel konstant ist, steht die nutzbare Wärme nur in einem beschränkten Zeitintervall zu Verfügung. Dieses Zeitintervall entspricht dem Zeitintervall, während dessen das Gasgemisch eine bestimmte Strecke der einen oder der mehreren nichtkatalytischen Zonen durchströmt und sich dabei entsprechend abkühlt.

Im Gegensatz zu herkömmlichen Dampfspaltverfahren existiert keine Kontrollmöglichkeit hinsichtlich der Konversion bzw. der Spaltbedingungen, beispielsweise durch eine Erhöhung oder Verringerung der externen Feuerung. Größere Massenstromänderungen in der Zusammensetzung des aus dem einen oder den mehreren katalytischen Zonen in die eine oder die mehreren nichtkatalytischen Zonen strömenden Gasgemischs, insbesondere dessen Gehalts an Ethan, haben somit einen hohen Einfluss auf die Konversion.

Zu hohe Konversionen besitzen starken Einfluss auf die Selektivität von Ethan zu Ethylen und führen verstärkt zur Bildung von Nebenprodukten. Im Gegenzug führen zu niedrige Konversionen zu einer Zunahme der Ethanmengen, die aus der einen oder den mehreren nichtkatalytischen Zonen abströmen und damit zu einer Massenstromzunahme in einem ggf. vorgesehenen Ethanrecycle. Die Problematik einer zu erreichenden Konversion verschärft sich hierdurch, bzw. kommt es hierdurch zur Notwendigkeit eines Ethanexports.

Massenstromänderungen des Ethans in dem aus der einen oder den mehreren katalytischen Zonen ausströmenden Gasgemischs sind z.B. möglich bei Änderungen in der Zusammensetzung von Erdgas, das als Einsatz in den einen oder die mehreren katalytischen Zonen verwendet wird, bei Änderungen in der Menge rückgeführten Ethans, beispielsweise im Rahmen von Anfahrprozeduren oder bei Störfällen, und bei Änderungen im katalytischen Verhalten des verwendeten Katalysators (beispielsweise aufgrund einer bekannten Alterung). Zudem können in der Anlagenauslegung zunächst nicht berücksichtigte Wärmeverluste dazu führen, dass aufgrund einer falsch ausgelegten Geometrie der einen oder der mehreren nichtkatalytischen Zonen gewünschte Zielkonversionen nicht erreicht werden. Insbesondere in letzterem Fall sind Änderungen an der einen oder den mehreren postkatalytischen Zonen im Nachgang nahezu unmöglich durchzuführen, da die eine oder die mehreren katalytischen Zonen, die eine oder die mehreren nichtkatalytischen Zonen und der sich anschließende Beginn der Kühlkette eine Einheit bilden bzw. fest platziert und dauerhaft miteinander verrohrt sind.

Insgesamt ist eine Erhöhung bzw. Verringerung der in der einen oder den mehreren postkatalytischen Zonen vorliegenden Temperaturen nur durch die Vorheizung des oder der in die eine oder die mehreren postkatalytischen Zonen eingespeisten Paraffine, beispielsweise von Ethan, bzw. durch die Einstellung der Austrittstemperatur aus der einen oder den mehreren katalytischen Zonen möglich. Beide Möglichkeiten zur Temperaturbeeinflussung sind jedoch begrenzt und haben nur minimalen Einfluss auf die Gesamtkonversion.

Die vorliegende Erfindung löst diese Probleme vollständig durch eine Kombination erfindungsgemäßer Maßnahmen. Hierzu werden die eine oder die mehreren nichtkatalytischen Zonen zunächst konservativ auslegt. Dies bedeutet, dass das durchströmte Volumen größer gewählt wird als dies für eine zu erreichende Konversion eines oder mehrerer Paraffine, insbesondere von Ethan, unter optimalen bzw. Auslegungsbedingungen erforderlich wäre. Ferner wird im Rahmen der vorliegenden Erfindung eine Möglichkeit zur wahlweisen Einspeisung eines oder mehrerer Paraffine, insbesondere von Ethan, an unterschiedlichen Positionen der einen oder der mehreren nichtkatalytischen Zonen geschaffen. Auf diese Weise können unterschiedliche Verweilzeiten eingestellt und auf diese Weise die Konversion des oder der Paraffine, insbesondere von Ethan zu Ethylen, kontrolliert werden.

Insgesamt schlägt die vorliegende Erfindung hierzu ein Verfahren zur Gewinnung eines oder mehrerer Olefine vor, bei dem ein Methan und Sauerstoff enthaltendes Einsatzgemisch einer oder mehreren katalytischen Zonen eines oder mehrerer Reaktoren zugeführt wird, wobei die eine oder die mehreren katalytischen Zonen einen Katalysator zur oxidativen Kopplung von Methan aufweisen. Zu weiteren Details entsprechender Katalysatoren bzw. entsprechend ausgebildeter Reaktoren sei auf den eingangs erläuterten Stand der Technik verwiesen. Der Fachmann wählt entsprechende Reaktoren bzw. Reaktortypen und die in diesen eingesetzten Katalysatoren nach Bedarf aus.

Wie bereits erwähnt, kommt die vorliegende Erfindung im Zusammenhang mit postkatalytischen Dampfspaltverfahren zum Einsatz, bei denen der einen oder den mehreren katalytischen Zonen eines oder mehrerer Reaktoren ein Gasgemisch entnommen und zumindest teilweise in eine oder mehrere nichtkatalytische Zonen des einen oder der mehreren Reaktoren oder eines oder mehrerer weiterer Reaktoren überführt wird. Details zu postkatalytischen Dampfspaltverfahren wurden bereits zuvor erläutert. Wie erwähnt, erfolgen in einem postkatalytischen Dampfspaltverfahren bzw. in einer oder mehreren entsprechenden Reaktionszonen thermische Dampfspaltschritte bzw. laufen dort entsprechende Reaktionen ab. Dies ergibt sich, wie erwähnt, aus der Temperatur des aus der oder den katalytischen Zonen des oder der Reaktoren ausströmenden Gasgemischs und des hier vorhandenen Wassers.

Wie grundsätzlich bekannt, werden auch im Rahmen der vorliegenden Erfindung in die eine oder mehrere nichtkatalytischen Zonen ein oder mehrere Paraffine eingespeist. Insbesondere kann es sich hierbei um Ethan handeln, es können jedoch auch andere Paraffine wie Propan, Butan und dergleichen entsprechend einer nichtkatalytischen Dampfspaltung unterworfen werden. Die nachfolgende Beschreibung der vorliegenden Erfindung in ihrer bevorzugten Ausführungsformen konzentriert sich dabei insbesondere auf Ethan, es sei jedoch zu verstehen gegeben, dass auch andere Paraffine in entsprechender Weise verwendet werden können.

Insbesondere kann im Rahmen der vorliegenden Erfindung jedoch auch Ethan alleine (zusätzlich zu dem aus der oder den katalytischen Zonen ausströmenden Gasgemisch) in eine oder mehrere nichtkatalytische Zonen eingespeist werden. Da bei der oxidativen Kopplung von Methan überwiegend kleine, kurzkettige Kohlenwasserstoffe wie Ethan bzw. Ethylen gebildet werden, ist es bei einer Einspeisung von Ethan in die eine oder die mehreren nichtkatalytischen Reaktionszonen eines entsprechenden Reaktors möglich, ein Produktspektrum zu erzeugen, das im Wesentlichen jenem der oxidativen Kopplung gleicht bzw. das derart beschaffen ist, dass nachgeordnete Trenneinrichtungen bzw. Trennschritte nicht in aufwendiger Weise angepasst werden müssen, beispielsweise an die Gewinnung längerkettige Kohlenwasserstoffe.

Grundsätzlich wird bzw. werden das oder die in die eine oder mehreren nichtkatalytischen Zonen eingespeisten Paraffine in der einen oder den mehreren nichtkatalytischen Zonen teilweise durch eine Dampfspaltreaktion zu einem oder mehreren Olefinen umgesetzt. Insbesondere bilden sich dabei die kettenlängengleichen Olefine der jeweils eingespeisten Paraffine, im Falle von Ethan also Ethylen. Neben dem oder den zusätzlich eingespeisten Paraffinen wird in der einen oder in den mehreren nichtkatalytischen Zonen auch das Ethan aus der oxidativen Kopplung von Methan umgesetzt.

Im Rahmen der vorliegenden Erfindung ist nun vorgesehen, dass das ein oder die mehreren Paraffine, also insbesondere das Ethan, an einer oder an mehreren von mehreren unterschiedlichen Einspeisepositionen, die während der Durchführung des Verfahrens in Abhängigkeit von einer vorgegebenen Konversion des einen oder der mehreren Paraffine ausgewählt wird oder werden, in die eine oder in die mehreren nichtkatalytischen Zonen eingespeist werden.

Die besonderen Vorteile dieses erfindungsgemäß vorgeschlagenen Verfahrens wurden bereits zuvor erläutert. Insbesondere kann durch eine entsprechende Auswahl von Einspeisepositionen in das durch die eine oder die mehreren nichtkatalytischen Zonen strömende Gasgemisch die Verweildauer bzw. die Eintrittstemperatur, die das eine oder die mehreren Paraffine ausgesetzt ist bzw. sind, in entsprechender Weise angepasst bzw. ausgewählt werden. Auf diese Weise kann auch schwankenden Verfahrensbedingungen, insbesondere schwankende Ethanmengen aus der oxidativen Kopplung von Methan, begegnet werden. Die Erfindung ermöglicht auf diese Weise eine sehr viel bessere Anpassung der Dampfspaltbedingungen als dies in postkatalytischen Dampfspaltverfahren gemäß dem Stand der Technik möglich ist. Zu weiteren Details sei auf die obigen Erläuterungen nochmals verwiesen.

Ist hier davon die Rede, dass das eine oder die mehreren Paraffine "an einer oder an mehreren von mehreren unterschiedlichen Einspeisepositionen" eingespeist werden, kann hier von sowohl umfasst sein, dass das oder die Paraffine an genau einer Einspeiseposition der unterschiedlichen Einspeisepositionen eingespeist wird bzw. werden, es kann jedoch auch vorgesehen sein, dass die Paraffine, gemeinsam oder getrennt voneinander, an mehreren unterschiedlichen Einspeisepositionen einzuspeisen. Wird beispielsweise Ethan an unterschiedlichen Einspeisepositionen eingespeist, so können die jeweils eingespeisten unterschiedlichen Ethananteile unterschiedlichen Dampfspaltbedingungen und damit unterschiedlichen Konversionen ausgesetzt werden. Auf diese Weise ist eine besonders günstige Anpassung oder auch ein Übergang zwischen unterschiedlichen Einspeisemodalitäten ohne abrupte Änderungen, d.h. graduell, möglich.

Ist im Rahmen dieser Anmeldung von "unterschiedlichen Einspeisepositionen" die Rede, sei dabei insbesondere unterschiedliche axiale Positionen in einem oder mehreren nichtkatalytischen Zonen des oder der verwendeten Reaktoren verstanden. Wie erläutert, sind derartige nichtkatalytische Zonen insbesondere rohrförmig bzw. allgemeiner zylindrisch ausgebildet und weisen daher eine Rohr- bzw. Zylinderachse auf. Die "unterschiedlichen Einspeisepositionen" entsprechen dabei unterschiedlichen axialen Positionen entlang der Rohr- bzw. Zylinderachse. Das aus der den katalytischen Zonen aus- und in die oder die eine oder die mehrere nichtkatalytischen Zonen einströmende Gasgemisch aus der oxidativen Kopplung von Methan strömt durch die eine oder die mehreren nichtkatalytischen Zonen im Wesentlichen in einer Richtung, die der Rohr- bzw. Zylinderachse entspricht. Das oder die zusätzlich an den unterschiedlichen Positionen eingespeisten Paraffine werden insbesondere mit diesem Gasgemisch mitgerissen und verweilt bzw. verweilen auf diese Weise unterschiedlich lange in der jeweiligen Reaktionszone.

Wie bereits teilweise zuvor erläutert, können im Rahmen der vorliegenden Erfindung insbesondere ein oder mehrere Reaktoren mit einer oder mehreren nichtkatalytischen Zonen verwendet werden, wobei die eine oder die mehreren nichtkatalytischen Zonen jeweils mehrere Einspeiseleitungen aufweisen, die an den mehreren unterschiedlichen Einspeisepositionen in die eine oder in die mehreren nichtkatalytischen Zonen münden. Die eine oder die mehreren Einspeisepositionen wird bzw. werden dabei zur Einspeisung des einen oder der mehreren Paraffine ausgewählt, wobei die Einspeiseleitungen je nach der gewünschter Einspeiseposition oder nach den gewünschten Einspeisepositionen mit dem einen oder den mehreren Paraffinen beaufschlagt wird oder werden. Wie bereits erläutert, kann auch eine gleichzeitige Einspeisung von unterschiedlichen Anteilen des einen oder der mehreren Paraffinen an unterschiedlichen Einspeisepositionen erfolgen, so dass auch ein Teil entsprechender Einspeiseleitungen mit dem einen oder den mehreren Paraffinen beaufschlagt werden kann, ein entsprechender anderer Anteil jedoch vorzugsweise nicht oder in geringerem Umfang.

Zur Vermeidung von Verwirbelungen in der einen oder in den mehreren nichtkatalytischen Zonen, die, wie erwähnt, insbesondere rohrförmig ausgebildet ist oder sind, kann gemäß einer besonders bevorzugten Ausgestaltung der vorliegenden Erfindung vorgesehen sein, an der einen oder an den mehreren Einspeisepositionen eine oder mehrere Einspeiselanzen bereitzustellen, die an der einen oder an den mehreren Einspeisepositionen in die eine oder in die mehreren nichtkatalytischen Zonen eingebracht, beispielsweise eingeschoben, werden können. Auch ein dauerhafter Verbleib entsprechender Einspeiselanzen ist grundsätzlich möglich, wobei jedoch in diesem Fall auf eine entsprechende Temperatur- bzw. Korrosionfestigkeit in dem heißen Gasgemisch zu achten ist.

Vorteilhafterweise sind dabei die Einspeiselanzen, insbesondere mehrfach und kreisförmig, um die eine oder um die mehreren nichtkatalytischen Zonen verteilt angeordnet. Auf diese Weise wird eine Einspeisesymmetrie erzielt, die Verwirbelungseffekte und damit unreproduzierbare Verfahrensbedingungen bzw. unterschiedliche Verweildauern weiter verringert.

Gemäß einer besonders bevorzugten Ausführungsform der vorliegenden Erfindung können, wie erwähnt, die eine oder die mehreren Einspeiselanzen an der einen oder an den mehreren Einspeisepositionen in die eine oder in die mehreren nichtkatalytischen Zonen eingeschoben werden. Auf diese Weise kann eine Einbringung entsprechender Einspeiselanzen nur dann erfolgen, wenn dies auf verfahrenstechnischen Gründen erforderlich ist. Zur anderen Zeiträumen können diese entsprechend geschont werden, in dem sie nicht den in der oder den nichtkatalytischen Zonen vorliegenden Hochtemperaturbedingungen mit entsprechender Korrosionsgefahr ausgesetzt werden.

Wie bereits zuvor erwähnt, kann die Wahl der einen oder der mehreren Einspeisepositionen während der Durchführung des Verfahrens insbesondere dynamisch erfolgen, so dass, mit anderen Worten, die eine oder die mehreren Einspeisepositionen während der Durchführung des Verfahrens in Abhängigkeit von einer vorgegebenen Konversion des einen oder der mehreren Paraffine jeweils verändert und angepasst werden kann bzw. können.

Die vorliegende Erfindung erstreckt sich auch auf eine Anlage zur Gewinnung eines oder mehrerer Olefine, wobei die Anlage einen oder mehrere Reaktoren mit einer oder mit mehreren katalytischen Zonen und mit einer oder mit mehreren nichtkatalytischen Zonen aufweist. Die Anlage ist dafür eingerichtet, ein Methan und Sauerstoff enthaltendes Einsatzgemisch der einen oder der mehreren katalytischen Zonen zuzuführen, der einen oder den mehreren katalytischen Zonen ein Gasgemisch zu entnehmen, das Gasgemisch zumindest teilweise in die eine oder in die mehreren nichtkatalytischen Zonen zu überführen, in die eine oder in die mehreren nichtkatalytischen Zonen ein oder mehrere Paraffine einzuspeisen, und das eine oder die mehreren in die eine oder in die mehreren nichtkatalytischen Zonen eingespeisten Paraffine in der einen oder mehreren nichtkatalytischen Zonen teilweise durch eine Dampfspaltreaktion zu einem oder zu mehreren Olefinen umzusetzen.

Erfindungsgemäß sind Einspeisemittel vorgesehen, die dafür eingerichtet sind, das eine oder die mehreren Paraffine in eine oder an mehreren von mehreren unterschiedlichen Einspeisepositionen, die während der Durchführung des Verfahrens von Abhängigkeit von einer vorgegebenen Konversion des einen oder der mehreren Paraffine auswählbar ist oder auswählbar sind, in die eine oder in die mehrere nichtkatalytischen Zonen einzuspeisen. Zu weiteren Merkmalen und Vorteilen der erfindungsgemäßen Anlage sei auf die obigen Erläuterungen verwiesen.

Insbesondere zeichnet sich eine entsprechende Anlage dadurch aus, dass die Einspeisemittel mehrere Einspeiseleitungen umfassen, die an den mehreren unterschiedlichen Einspeisepositionen in die eine oder in die mehreren nichtkatalytischen Zonen münden. Diese Einspeiseleitungen sind gemäß einer besonders bevorzugten Ausführungsform der vorliegenden Erfindung insbesondere wahlweise mit dem oder den eingespeisten Paraffinen beaufschlagbar.

Ferner sind mit den Einspeiseleitungen gemäß einer besonders bevorzugten Ausführungsform der Erfindung zur Vermeidung von Verwirbelungen bei der Einspeisung des einen oder mehreren Paraffine insbesondere mit Einspeiselanzen gekoppelt, die insbesondere in die eine oder in die mehreren nichtkatalytischen Zonen einschiebbar sind, wie ebenfalls erläutert.

Zu weiteren Merkmalen und Vorteilen der erfindungsgemäßen Anlage, die vorteilhafterweise zur Durchführung eines Verfahrens eingerichtet ist, wie es zuvor in unterschiedlichen Ausgestaltungen erläutert wurde, sei auf die obigen Ausführungen ausdrücklich verwiesen.

Die vorliegende Erfindung wird nachfolgend unter Bezugnahme auf die beigefügten Zeichnungen näher erläutert, welche eine bevorzugte Ausführungsform der Erfindung und weitere Details der vorliegenden Erfindung veranschaulichen.

### Kurze Beschreibung der Zeichnungen

Figur 1 veranschaulicht eine Anlage gemäß einer besonders bevorzugten Ausführungsform der Erfindung in schematischer Teildarstellung.
Figur 2 veranschaulicht eine Abhängigkeit zwischen einer Konversion und einer Verweildauer in Form eines Diagramms.
Figur 3A veranschaulicht eine Konversion in Abhängigkeit von einem rückgeführten Ethananteil bei niedriger Verweilzeit in Form eines Diagramms
Figur 3B veranschaulicht eine Konversion in Abhängigkeit von einem rückgeführten Ethananteil bei hoher Verweilzeit in Form eines Diagramms.
Figur 4 veranschaulicht eine Bildung von Nebenprodukten in Abhängigkeit von einer vorgegebenen Konversion gemäß einer Ausführungsform der vorliegenden Erfindung.

### Ausführliche Beschreibung der Zeichnungen

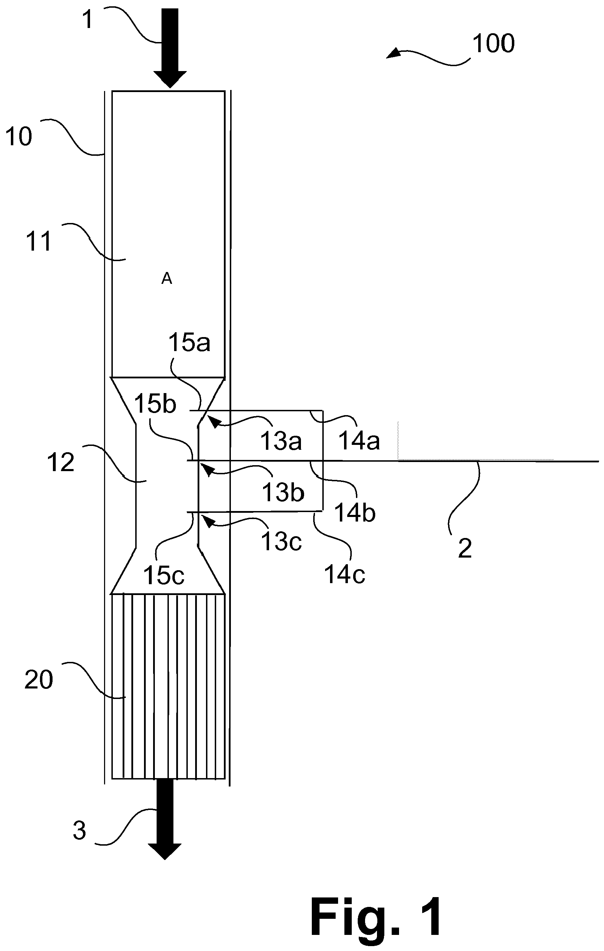

In Figur 1 ist eine Anlage gemäß einer besonders bevorzugten Ausführungsform der vorliegenden Erfindung veranschaulicht und insgesamt mit 100 bezeichnet. Die Darstellung der Figur 1 ist dabei stark vereinfacht und es sind nur Teile einer entsprechenden Anlage 100 dargestellt.

Wesentliche Komponente der Anlage 100 ist ein Reaktor 10, der eine katalytische Zone 11 und eine nichtkatalytische Zone 12 aufweist. In der katalytische Zone 11 ist dabei ein zur oxidativen Kopplung von Methan eingerichteter bzw. befähigter Katalysator vorgesehen. Der katalytischen Zone 11 des Reaktors 10 wird ein Methan und Sauerstoff enthaltendes Einsatzgemisch 1 zugeführt. In der katalytischen Zone 11 des Reaktors 10 wird eine oxidative Kopplung von Methan durchgeführt, so dass zumindest ein Teil des in dem Einsatzgemisch enthaltenen Methans und Sauerstoff miteinander umgesetzt werden.

Aus der katalytischen Zone 11 strömt daher ein Gasgemisch in die nichtkatalytische Zone 12, das nicht umgesetzte Edukte und bei der oxidativen Kopplung von Methan gebildete Produkte umfasst. In die nichtkatalytische Zone 12 des Reaktors 10 wird bzw. werden ferner ein oder mehrere Paraffine 2, insbesondere Ethan, eingespeist. Ein aus der nichtkatalytischen Zone 12 ausströmendes Gasgemisch wird insbesondere einem Quench bzw. Kühlbereich 20 zugeführt, in dem das Gasgemisch beispielsweise unter Verwendung eines Transferleitungskühlers (engl. Transfer Line Heat Exchanger, TLE) abgekühlt wird. Den Kühlbereich 20 verlässt ein Gasgemisch 3, das einer sich anschließenden Produktaufbereitung bzw. Produkttrennung zugeführt werden kann.

Im Rahmen der in Figur 1 veranschaulichten Ausgestaltung der vorliegenden Erfindung ist nun vorgesehen, dass oder die Paraffine 2 unterschiedlichen, wählbaren Positionen 13a, 13b und 13c in die nichtkatalytische Zone 12 einzuspeisen. Wie erwähnt, kann dabei eine Einspeisung an mehreren Positionen gleichzeitig erfolgen. An den Einspeisepositionen 13a, 13b und 13c münden hierzu entsprechende Einspeiseleitungen 14a, 14b und 14c. Insbesondere können an den Einspeisepositionen 13a, 13b und 13c Einspeiselanzen, wie hier stark vereinfacht mit 15a, 15b und 15c veranschaulicht, in die nichtkatalytische Zone 12 münden.

Im Rahmen der in Figur 1 veranschaulichten Ausführungsform der vorliegenden Erfindung können auf diese Weise die Einspeisepositionen 13a, 13b und 13c frei gewählt werden, insbesondere gemäß einer gewünschten Konversionsrate des oder der eingespeisten Paraffine 2. Durch eine entsprechende Einspeisung an einer oder mehreren unterschiedlichen Einspeisepositionen 13a, 13b, 13c kann insbesondere die Verweildauer des einen oder der mehreren Paraffine in der nichtkatalytischen Zone 12 eingestellt werden.

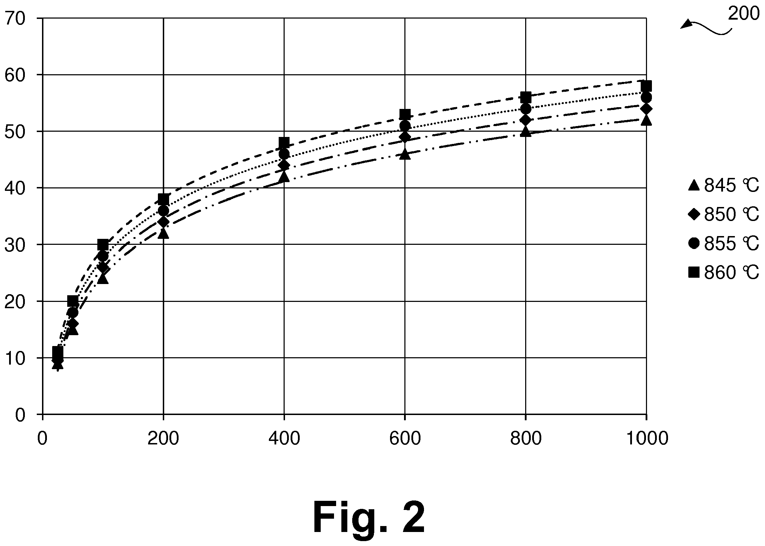

In Figur 2 ist eine Konversion in Abhängigkeit von einer Verweildauer in Form eines Diagramms 200 veranschaulicht. In dem Diagramm 200 ist die Verweildauer in Millisekunden auf der Abszisse gegenüber der Konversion von Ethan in Gewichtsprozent auf der Ordinate, bezogen auf den trockenen Anteil eines entsprechenden Gasgemischs, veranschaulicht. Hierbei sind vier unterschiedliche Abhängigkeiten für die angegebenen Eintrittstemperaturen von 845 °C, 850 °C, 855 °C und 860 °C veranschaulicht.

In einem in Figur 2 zugrundeliegenden Experiment wurde beispielsweise ein aus einer oxidativen Kopplung von Methan, d.h. einer katalytischen Zone, ausströmendes Gasgemisch mit zusätzlichem Ethan gemischt. In einem adiabaten Rohrreaktor, d.h. einer nichtkatalytischen Zone wurde das Ethan thermisch zu Ethylen umgesetzt. Die Mischtemperatur der beiden Ströme, d.h. des Gasgemischs aus der oxidativen Kopplung von Methan und des frisch zugesetzten Ethans betrug in einem Fall 845 °C. Zum Erreichen einer Zielkonversion von 50% wurden dabei ca. 800 Millisekunden benötigt. Unter der Annahme von vorhergegangenen vermeidbaren Temperaturverlusten von ca. 15 K im Reaktor, d.h. der katalytischen Zone, wäre eine Konversion innerhalb von 400 Millisekunden ebenso möglich. Im Idealfall, d.h. unter Vernachlässigung von Wärmeverlusten, geht man von einer nochmals geringeren Verweildauer von 250 bis 300 Millisekunden aus.

Unter den vorliegenden Bedingungen führt ein Temperaturverlust von 5 K zu einer Konversionseinbuße von ca. 2%, wie aus Figur 2 unmittelbar ersichtlich. Eine Kompensation einer derartigen Konversionseinbuße entspricht einer Verweilzeiterhöhung von ca. 80 Millisekunden. Ein Temperaturverlust von 5 K stromauf der postkatalytischen Dampfspaltung entspricht somit einer zusätzlich benötigten relativen Verweilzeit von 30 bis 35% bezogen auf den Idealfall. Durch den Einsatz der Erfindung, d.h. die Einspeisung an unterschiedlichen Einspeisepositionen, kann eine Reaktion auf entsprechende Abweichungen vom Idealfall erfolgen.

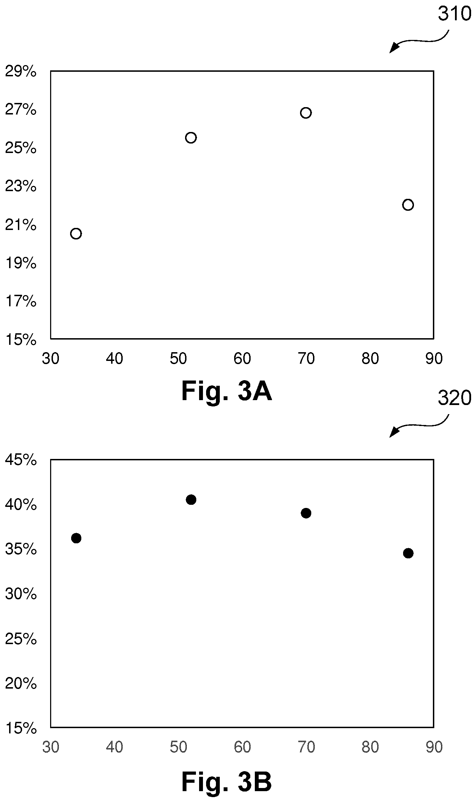

In Figur 3A ist eine Abhängigkeit der Konversion von einer rückgeführten Ethanmenge in Form eines Diagramms 310 veranschaulicht. Hierbei ist eine Menge an rückgeführtem Ethan in Kilogramm pro Stunde auf der Abszisse gegenüber einer Konversion in den gleichen Dimensionen wie gemäß der zuvor erläuterten Figur 2 auf der Ordinate dargestellt.

Das in Figur 3A zugrundeliegende Experiment dient zur Veranschaulichung des Effekts eines Überschusses bzw. eines Mangels an Ethan in einem entsprechenden Verfahren. Das Gasgemisch aus der oxidativen Kopplung von Methan liegt dabei bei einer Temperatur von 866 °C und einem Überdruck von 8 bar vor. Etwa 1.000 Kilogramm pro Stunde dieses Gasgemischs strömen aus der oder den nichtkatalytischen Zonen eines entsprechenden Reaktors aus. Es wurden unterschiedliche Mengen, d.h. 34 bis 84 kg pro Stunde Ethan, in die nichtkatalytische Zone eines entsprechenden Reaktors zugespeist. Je nach zugespeister Ethanmenge werden 27 bis 65 Kilogramm pro Stunde Ethan in dem ausströmenden Gasgemisch erhalten. Am Ende der nichtkatalytischen Zone liegt das Gasgemisch bei einer Temperatur von 812 °C vor.

Wie aus Figur 3B veranschaulicht, in der erneut Konversionen in Abhängigkeit von der rückgeführten Ethanmenge veranschaulicht sind, kann zum Ausgleich der niedrigen Konversion bei hoher Ethanmenge eine Verlängerung der Verweildauer durchgeführt werden. In dem der Figur 3B zugrunde liegenden Experiment konnten dabei erhöhte Konversionen von max. 40% erzielt werden. Während gemäß Figur 3A eine Verweildauer von 300 Millisekunden vorgegeben wurde, betrug diese gemäß Figur 3B ca. 1000 Millisekunden. Am Ende der nichtkatalytischen Zone beträgt die Temperatur des Gasgemischs daher 800 °C.

Ein Ausgleich kann auch in dem Sinne erfolgen, dass das Ethan aus zwei unterschiedlichen Höhen eingespeist wird, also an zwei unterschiedlichen Einspeisepositionen. Auf diese Weise kann eine Überkompensation von Konversionseinbußen verhindert werden.

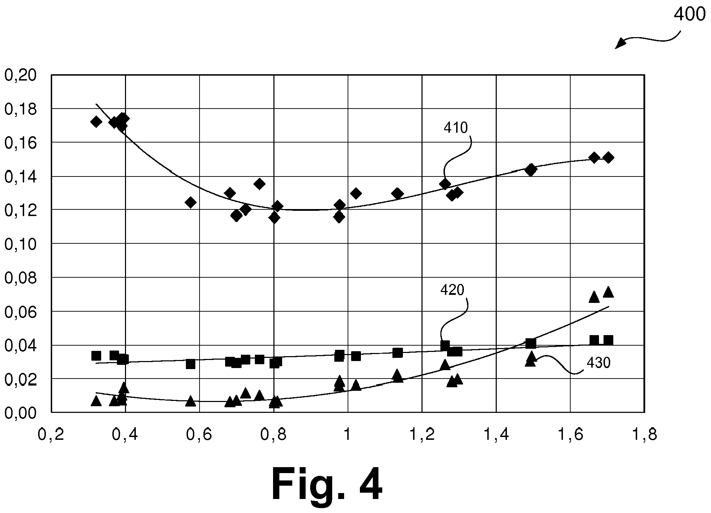

In Figur 4 ist die Bildung von Nebenprodukten in Abhängigkeit von der Ethylenzunahme innerhalb des postkatalytischen Dampfspaltens in Form eines Diagramms 400 veranschaulicht.

Mit zunehmender Konversion des Ethans, hier dargestellt als das Verhältnis zwischen Ethylen und Ethan aufgetragen auf der Abszisse, nehmen auch die Nebenprodukte aus der postkatalytischen Dampfspaltung zu. Auf der Ordinate aufgetragen sind typische Nebenprodukte in ihrem jeweiligen Verhältnis zum Hauptprodukt Ethylen. Hierbei sind Kohlenwasserstoffe mit drei Kohlenstoffatomen als Punkte bzw. Datenreihe 410, Kohlenwasserstoffe mit vier Kohlenstoffatomen als Datenreihe 420 und Benzol als Datenreihe 430 veranschaulicht.

Wie zu erkennen, steigen mit zunehmender Konversion des Ethans die Nebenprodukte in ihrer Konzentration an. Eine Selektivitätskontrolle kann somit mit der Erfindung beschrieben Verbesserung zur der Kontrolle der Konversion erreicht werden.

## Patentansprüche

1. Verfahren zur Gewinnung eines oder mehrerer Olefine, bei dem ein Methan und Sauerstoff enthaltendes Einsatzgemisch (1) einer oder mehreren katalytischen Zonen (11) eines oder mehrerer Reaktoren (10) zugeführt wird, wobei die eine oder die mehreren katalytischen Zonen (11) einen Katalysator zur oxidativen Kopplung von Methan aufweisen, wobei der einen oder den mehreren katalytischen Zonen (11) ein Gasgemisch entnommen wird und zumindest teilweise in eine oder mehrere nichtkatalytische Zonen (12) des einen oder der mehreren Reaktoren (10) überführt wird, wobei in die eine oder die mehrere nichtkatalytischen Zonen (12) ein oder mehrere Paraffine (2) eingespeist werden, und wobei das eine oder die mehreren in die eine oder in die mehreren nichtkatalytischen Zonen (12) eingespeisten Paraffine (2) in der einen oder in den mehreren nichtkatalytischen Zonen (12) teilweise durch eine Dampfspaltreaktion zu einem oder mehreren Olefinen umgesetzt werden, **dadurch gekennzeichnet, dass** das eine oder die mehreren Paraffine (2) an einer oder an mehreren von mehreren unterschiedlichen Einspeisepositionen (13a, 13b, 13c), die während der Durchführung des Verfahrens in Abhängigkeit von einer vorgegebenen Konversion des einen oder der mehreren Paraffine (2) ausgewählt wird oder werden, in die eine oder die mehreren nichtkatalytischen Zonen (12) eingespeist werden.

2. Verfahren nach Anspruch 1, bei dem Ethan als das Paraffin (2) verwendet wird.

3. Verfahren nach Anspruch 1 oder 2, bei dem ein oder mehrere Reaktoren (10) mit einer oder mehreren nichtkatalytischen Zonen (12) verwendet werden, wobei die eine oder die mehreren nichtkatalytischen Zonen (12) mehrere Einspeiseleitungen (14a, 14b, 14c) aufweist oder aufweisen, die an den mehreren unterschiedlichen Einspeisepositionen (13a, 13b, 13c) in die eine oder in die mehreren nichtkatalytischen Zonen (12) münden, und wobei die eine oder die mehreren Einspeisepositionen (13a, 13b, 13c) ausgewählt wird oder werden, indem die Einspeiseleitungen (14a, 14b, 14c) mit dem einen oder mit den mehreren Paraffinen (2) beaufschlagt wird oder werden.

4. Verfahren nach einem der vorstehenden Ansprüche, bei dem zur Einspeisung des einen oder den mehreren Paraffine (2) an der einen oder an den mehreren Einspeisepositionen (13a, 13b, 13c) eine oder mehrere Einspeiselanzen (15a, 15b, 15c) an der einen oder an den mehreren Einspeisepositionen (13a, 13b, 13c) in die eine oder die mehreren nichtkatalytischen Zonen (12) eingebracht werden.

5. Verfahren nach Anspruch 4, bei dem die Einspeiselanzen (13a, 13b, 13c) um die eine oder die mehreren nichtkatalytischen Zonen (12) angeordnet sind.

6. Verfahren nach Anspruch 4 oder 5, bei dem die eine oder die mehreren Einspeiselanzen (15a, 15b, 15c) an der einen oder an den mehreren Einspeisepositionen (13a, 13b, 13c) in die eine oder die mehreren nichtkatalytischen Zonen (12) eingeschoben werden.

7. Verfahren nach einem der vorstehenden Ansprüche, bei dem die eine oder die mehreren Einspeisepositionen (13a, 13b, 13c) während der Durchführung des Verfahrens in Abhängigkeit von einer vorgegebenen Konversion des einen oder der mehreren Paraffine (2) verändert werden.

8. Anlage (100) zur Gewinnung eines oder mehrerer Olefine, wobei die Anlage (100) einen oder mehrere Reaktoren (10) mit einer oder mit mehreren katalytischen Zonen (11) und mit einer oder mit mehreren nichtkatalytischen Zonen (12) aufweist, und wobei die Anlage (100) dafür eingerichtet ist, ein Methan und Sauerstoff enthaltendes Einsatzgemisch (1) der einen oder den mehreren katalytischen Zonen (11) zuzuführen, der einen oder den mehreren katalytischen Zonen (11) ein Gasgemisch zu entnehmen, das Gasgemisch zumindest teilweise in die eine oder in die mehreren nichtkatalytische Zonen (12) zu überführen, in die eine oder die mehreren nichtkatalytischen Zonen (12) ein oder mehrere Paraffine (2) einzuspeisen, und das eine oder die mehreren in die eine oder die mehreren nichtkatalytischen Zonen (12) eingespeisten Paraffine (2) in der einen oder den mehreren nichtkatalytischen Zonen (12) teilweise durch eine Dampfspaltreaktion zu einem oder mehreren Olefinen umzusetzen, **dadurch gekennzeichnet, dass** Einspeisemittel vorgesehen sind, die dafür eingerichtet sind, das eine oder die mehreren Paraffine (2) an einer oder an mehreren von mehreren unterschiedlichen Einspeisepositionen (13a, 13b, 13c), die während der Durchführung des Verfahrens in Abhängigkeit von einer vorgegebenen Konversion des einen oder der mehreren Paraffine (2) auswählbar ist oder auswählbar sind, in die eine oder in die mehreren nichtkatalytischen Zonen (12) einzuspeisen.

9. Anlage (100) nach Anspruch 8, bei der die Einspeisemittel mehrere Einspeiseleitungen (14a, 14b, 14c) umfassen, die an den mehreren unterschiedlichen Einspeisepositionen (13a, 13b, 13c) in die eine oder in die mehreren nichtkatalytischen Zonen (12) münden.

10. Anlage (100) nach Anspruch 9, bei der die Einspeiseleitungen (14a, 14b, 14c) wahlweise mit dem oder den Paraffinen (2) beaufschlagbar sind.

11. Anlage (100) nach Anspruch 8 oder 9, bei dem die Einspeiseleitungen (14a, 14b, 14c) mit Einspeiselanzen (15a, 15b, 15c) gekoppelt sind.

12. Anlage (100) nach Anspruch 11, bei dem die Einspeiselanzen (15a, 15b, 15c) an den mehreren unterschiedlichen Einspeisepositionen (13a, 13b, 13c) in die eine oder in die mehreren nichtkatalytischen Zonen (12) einschiebbar sind.

13. Anlage (100), die zur Durchführung eines Verfahrens nach einem der Ansprüche 1 bis 7 eingerichtet ist.
